# EUROPEAN PATENT SPECIFICATION

(11) **EP 2 860 184 B1**
(45) Date of publication and mention of the grant of the patent: **29.08.2018**
(21) Application number: 13004844.0
(22) Date of filing: 09.10.2013
(51) Int. Cl.: C07F 9/6561, A61K 31/675

(54) **Dihydrogenphosphate salt of Tenofovir disoproxil**
Dihydrogenphosphatsalz von Tenofovirdisoproxil
Sel dihydrogénophosphate du Ténofovir disoproxil

(43) Date of publication of application: 15.04.2015
(73) Proprietor: Zentiva, k.s., 102 37 Praha 10 (CZ)
(72) Inventor: Holan, Jan, 747 68 Kyjovice (CZ); Ridvan, Ludek, 102 00 Praha 10 (CZ); Zapadlo, Michal, 549 41 Cerveny Kostelec (CZ); Dammer, Ondrej, 253 01 Hostivice (CZ); Beranek, Josef, 160 00 Praha 6 (CZ); Kral, Vladimir, 120 00 Praha 2 (CZ)
(74) Representative: Jirotkova, Ivana

(56) References cited:
- WO-A1-2013/132314
- CN-B- 101 781 334

## Description

### Filed of the Invention

The present invention relates to the crystalline form of Tenofovir disoproxil dihydrogenphosphate, improved process for its preparation and its use in pharmaceutical compositions.

Tenofovir disoproxil is chemically known as 9-[-2-(R)-[[bis[[(isopropoxycarbonyl)oxy] methoxy]phosphinoyl]methoxy] propyl] adenine represented by the following structure:

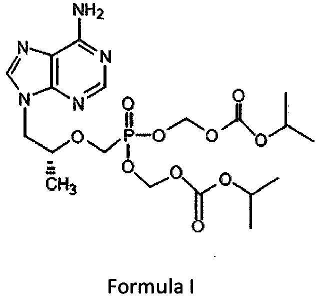

Tenofovir disoproxil, used in drug products (e.g. Truvada® or Atripla®) in the form of fumarate salt with Formula II, is a highly potent antiviral agent, particularly for the therapy or prophylaxis of retroviral infections and belongs to a class of drugs called Nucleotide Reverse Transcriptase Inhibitors (NRTI) which block reverse transcriptase, an enzyme crucial to viral production in HIV-infected people.

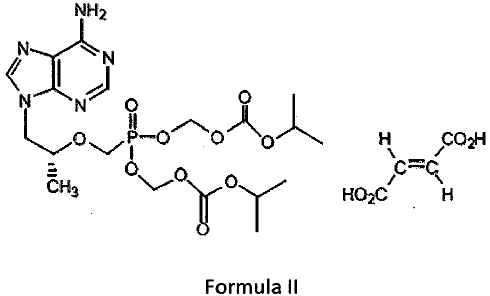

### Background of the Invention

The following procedures of Tenofovir disoproxil preparation are known from patents WO9804569**,** EP0915894**,** EP0998480**,** WO9905150**,** US5935946 (GILEAD).

Patents WO2007013086 (HETERO), WO2009130437 (CIPLA) and WO2008007392**,** EP2046792A2 (Mylan/MATRIX) describe polymorphic forms and amorphous forms of Tenofovir disoproxil fumarate (Formula II) and hemifumarate (Formula III).

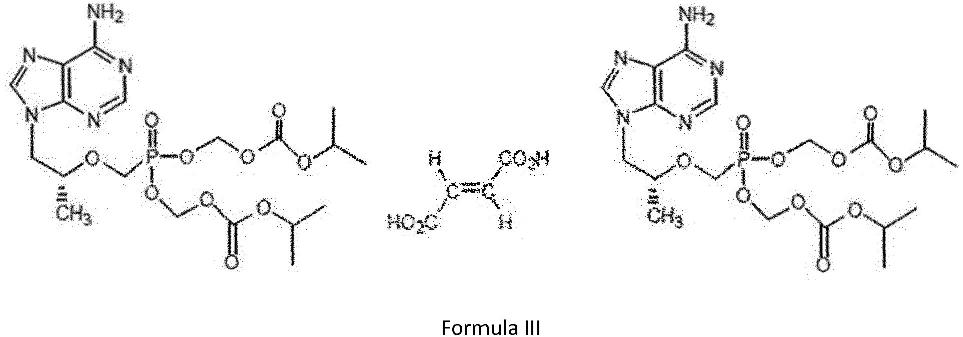

Other polymorphic forms of Tenofovir disoproxil fumarate and cocrystal with fumaric acid can be found in the patents WO2008140302**,** WO2009064174**,** WO2008143500(ULTIMORPHIX). Tenofovir disoproxil salts describe the patents WO2009074351**,** WO2010142761A1(ULTIMORPHIX). Tenofovir disoproxil dihydrogenphosphate and his preparation by dissolving tenofovir disoproxil in hot isopropanol at 50°C, adjusting to pH 2-3 with phosphoric acid and cooling to 0°C is described in the patent CN101781334 (Fujian Consunter Pharmaceutical Co., LTD). The product shows the following characteristic reflections in the XRPD measured by CuKα emission: 5.6; 7.62; 12.36; 13.56; 16.34; 22.38 and 25.36°2θ.

In the patent WO2008143500 (ULTIMORPHIX) is described that mixtures of solid forms were found in several commercially available products containing Tenofovir disoproxil fumarate. The authors found that Tenofovir disoproxil fumarate (Formula II) converts into Tenofovir disoproxil hemifumarate (Formula III) in the presence of increased humidity and/or temperature. Authors of WO2008143500 conclude that "the solid form currently used in the marketed product is not stable or at least has a reduced stability".

It is obvious that there is a need for Tenofovir disoproxil solid form with required chemical purity and physical stability suitable for manufacture of stable drug product.

### Summary of Invention

The present invention provides a method of preparation of Tenofovir disoproxil dihydrogenphosphate of formula IV 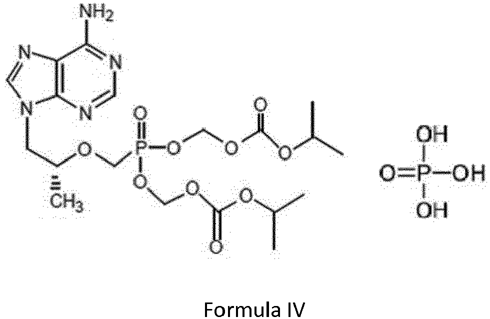 showing the following characteristic reflections in the XRPD measured by CuKα emission 5,6; 7,7; 12,4; 13,6; 16,4; 22,4 a 25,4 ± 0,2 ° 2θ, wherein Tenofovir disoproxil fumarate is dissolved in an organic solvent selected from C1-C4 alkylalcohols or toluene at temperature 55-60 °C, the solution is seeded by the product, phosphoric acid is added dropwise into the solution, the resulting reaction mixture is cooled down to 20-45 °C by the cooling ramp 0.3333 K/min and crystalline Tenofovir disoproxil dihydrogenphosphate is isolated.

Preferably, Tenofovir disoproxil fumarate is dissolved in isopropanol at 60 °C, the solution is seeded by the product (5-10% of an amount of starting material), 1.1-5 equivalents of phosphoric acid (85 %) is added dropwise into the solution, the resulting reaction mixture is cooled down to 20 °C by the cooling ramp 0.3333 K/min and crystalline Tenofovir disoproxil dihydrogenphosphate is isolated.

It was found out that the cooling profile as well as final crystallization temperature plays a key role in the product purity and yield. Both methods of preparation according to the invention lead to the product with high chemical purity and defined particle size distribution. Excellent physico-chemical properties (stability and solubility) make this salt particularly suitable for the preparation of a medicinal product.

Tenofovir disoproxil dihydrogenphosphate can be obtained with chemical purity at least 99.0 % or 99.5 %. A special attention is paid to fumaric acid impurity in the process starting from Tenofovir disoproxil fumarate. The manufacturing procedure according to the present invention allows to obtain Tenofovir disoproxil dihydrogenphosphate with the content of fumaric acid less than 0.10 % in a single crystallization step. That's a significant improvement with regard to the state of the art. Because the experiments performed according to procedure described in patent CN101781334 gave the product (Tenofovir disoproxil phosphate) with relatively high content of fumaric acid (0.5 % or more).

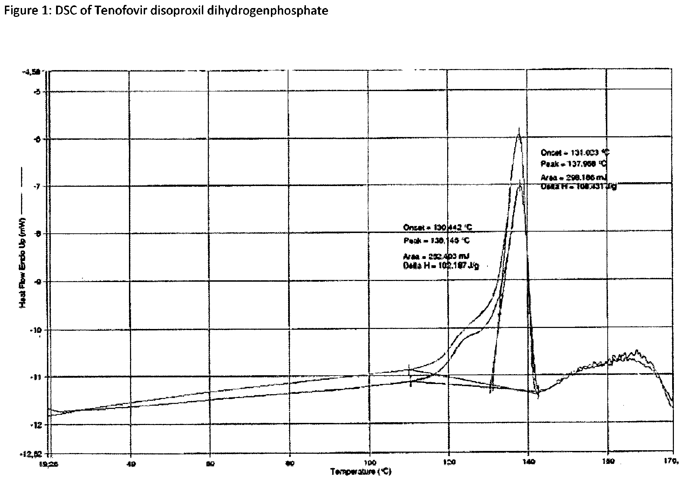

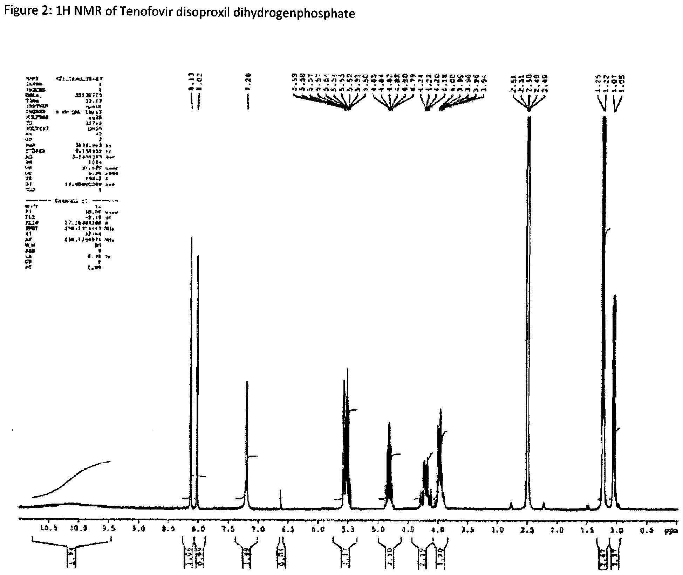

Tenofovir disoproxil dihydrogenphosphate is a white powder with melting point about 138 °C (Figure 1). The ratio of Tenofovir disoproxil and phosphoric acid is 1:1 in the molecule. The ratio was confirmed by 1H NMR (Figure 2).

Tenofovir disoproxil dihydrogenphosphate is a stable form characterized by the reflection in RTG powder data: 5.6; 7.7; 12.4; 13.6; 16.4; 22.4 and 25.4 ± 0.2 ° 2θ (Table 1), that was measured by using of CuKα emission.

**Table 1: XRPD - characteristic diffraction peaks corresponding Tenofovir disoproxil dihydrogenphosphate**

| Pos. [°2Th.] | d-spacing [A] | Rel. Int. [%] |
|---|---|---|
| 5,65 | 15,636 | 100,0 |
| 7,67 | 11,517 | 63,0 |
| 9,53 | 9,275 | 17,2 |
| 12,42 | 7,124 | 48,9 |
| 13,61 | 6,501 | 36,9 |
| 14,50 | 6,105 | 17,8 |
| 15,57 | 5,688 | 19,5 |
| 15,84 | 5,592 | 26,8 |
| 16,41 | 5,398 | 45,1 |
| 16,78 | 5,281 | 18,8 |
| 17,02 | 5,206 | 30,3 |
| 18,40 | 4,819 | 6,7 |
| 18,67 | 4,749 | 10,7 |
| 19,10 | 4,642 | 7,6 |
| 20,40 | 4,350 | 20,0 |
| 21,22 | 4,184 | 25,0 |
| 22,42 | 3,962 | 67,7 |
| 22,75 | 3,905 | 17,5 |
| 22,97 | 3,869 | 13,5 |
| 24,06 | 3,696 | 22,7 |
| 25,01 | 3,558 | 21,1 |
| 25,40 | 3,503 | 29,4 |
| 26,67 | 3,340 | 5,6 |
| 27,52 | 3,239 | 4,3 |
| 28,54 | 3,125 | 4,3 |
| 29,48 | 3,027 | 2,6 |
| 30,23 | 2,954 | 2,8 |
| 30,73 | 2,907 | 2,4 |
| 32,14 | 2,783 | 12,7 |

Physico-chemical properties (stability and solubility) of Tenofovir disoproxil dihydrogenphosphate were compared with other known salts (Tenofovir disoproxil fumarate, citrate, succinate, L-tartrate, D-tartrate, maleate) prepared according to published procedures. Tenofovir disoproxil dihydrogenphosphate is the most stable salt of Tenofovir disoproxil, as shown in Table 2.

**Table 2: Chemical stability of Tenofovir disoproxil and its salts (stress conditions: 80°C for 46 hours)**

| **Compound** | **Starting HPLC purity (%)** | **HPLC purity (%) after the stress test** |
|---|---|---|
| Tenofovir disoproxil fumarate | 99.81 | 4.08 |
| Tenofovir disoproxil (free base) | 95.66 | 6.13 |
| Tenofovir disoproxil L-tartrate | 98.10 | 21.12 |
| Tenofovir disoproxil D-tartrate | 99.66 | 25.51 |
| Tenofovir disoproxil succinate | 99.04 | 54.16 |
| Tenofovir disoproxil dihydrogenphosphate | 99.66 | 97.69 |

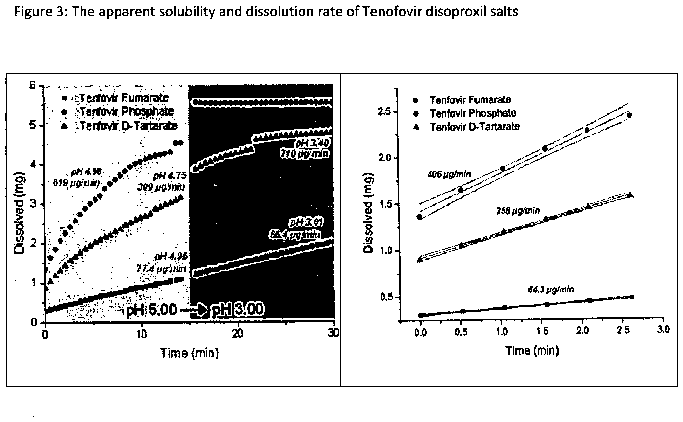

The active substance solubility is important for bioavailability of the drug. Solubility of Tenofovir disoproxil dihydrogenphosphate is superior to other salts, including Tenofovir disoproxil fumarate (Figure 3).

We analysed different batches of commercially available drug products (Truvada® and Atripla®) containg Tenofovir disoproxil fumarate (Formula II) by XRPD and we found various amount of Tenofovir disoproxil hemifumarate (Formula III). Moreover, we observed partial the conversion (about 30 %) of Tenofovir disoproxil fumarate (Formula II) in Tenofovir disoproxil hemifumarate (Formula III) during our formulation trials using wet granulation process. On the other hand, we observed no changes in API solid form when using Tenofovir disoproxil dihydrogenphosphate for manufacturing of a drug product.

Different solid forms generally differ in physico-chemical properties, such as solubility or chemical stability. This may influence not only shelf-life of the drug product but also bioavailability and thus safety and efficacy. Physico-chemical properties of Tenofovir disoproxil dihydrogenphosphate makes this salt suitable for use in the preparation of pharmaceutical compositions.

### Characterization methods

### XRPD (X-Ray Powder Diffractometry)

Diffraction patterns were obtained with laboratory X'PERT PRO MPD PANalytical diffractometer, radiation used CuKα (λ = 1.542Å).

Generator settings: excitation voltage 45 kV, anodic current 40 mA.

Scan description: scan type - gonio, measurement range 2 - 40º 2θ, step size 0.01º 2θ, step time: 0.5 s. Samples were measured as received on Si plate (zero background holder). Incident beam optics: programmable divergence slits (irradiated length 10 mm), 10 mm mask, 1/4º anti-scatter fixed slit, 0.02 rad Soller slits.

Diffracted beam optics: X'Celerator detector, scanning mode, active length 2.122º, 0.02 rad Soller slits, anti-scatter slit 5.0 mm, Ni filter.

### HPLC (high-performance liquid chromatography)

Column: Zorbax Eclipse XDB C18 RRHD, 100 x 3.0 mm (I.D.) x 1.8 um (Agilent), Run Time: 11.5 min., Injection Volume: 2 µl, Mobile Phase: MFA: 10mM K2HPO4 pH=7,0; MFB: ACN; MFA: 90-40-40-90; t: 0-7-9-9,5; F: 0,5 ml/min; 35°C

### H¹-NMR

For ¹H NMR spectra the Bruker NMR spectrometer AVANCE 500 MHz and DMSO as solvent were used. The stoichiometry of salts were determinated from integrals of corresponding signals of API and coformer.

### DSC

DSC curves were recorded using a Perkin Elmer Pyris 1 differential scanning calorimetr. For the measurements approximately 2 - 6 mg of sample were placed in aluminium pans, accurately weighed and hermatically closed with perforation lids. Prior to measurement the lids were pierced resulting in approximately 1.5 mm pin holes. The samples were then heated under a flow of nirtogen of about 100 mL/min using heating rates of usually 10 K/min.

### Examples

### Example 1:

### Preparation of Tenofovir disoproxil dihydrogenphosphate from Tenofovir disoproxil base

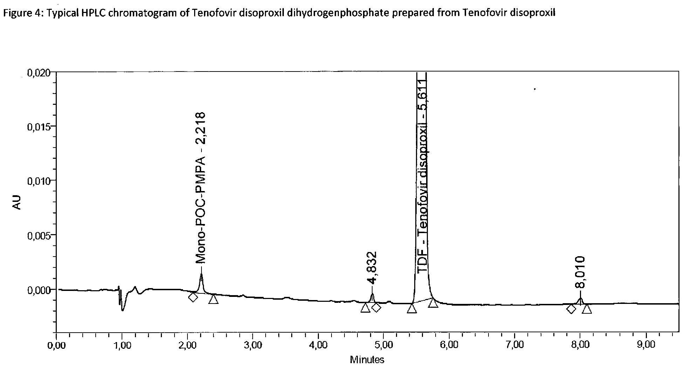

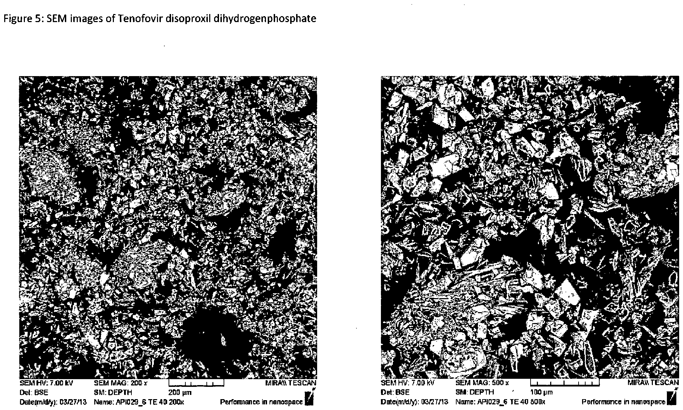

Tenofovir disoproxil base (2.78 g) was dissolved in isopropanol (90 ml) in the 100 ml vessel (Easy Max) at 60 °C. Afterwards 1 equivalent of phosphoric acid (0.33 ml 85 %) was added dropwise into the vessel. After 90 min the suspension was cooled down to 0 °C by the cooling ramp 0.1 K/min. Tenofovir disoproxil phosphate was filtered, washed up two times by isopropanol (2x30 ml) and dried up by room temperature overnight (16 hours). The isolated yield of crystallization was 80 % (HPLC: 99.56 %). Chromatographic result can be found in the appendix (Figure 4). Particle size distribution was obtained by scanning electron microscope. Most of the particles were smaller than 100 µm (Figure 5).

### Example 2:

### Preparation of Tenofovir disoproxil dihydrogenphosphate from Tenofovir disoproxil fumarate

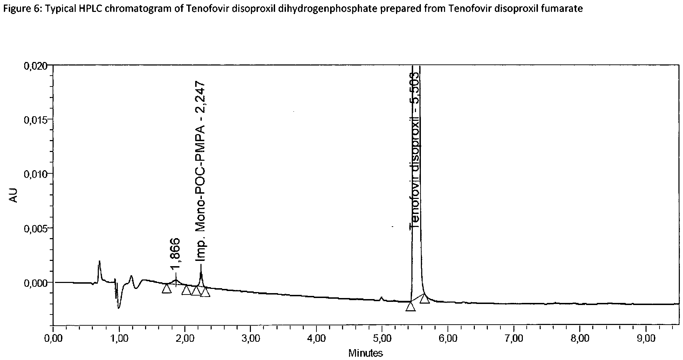

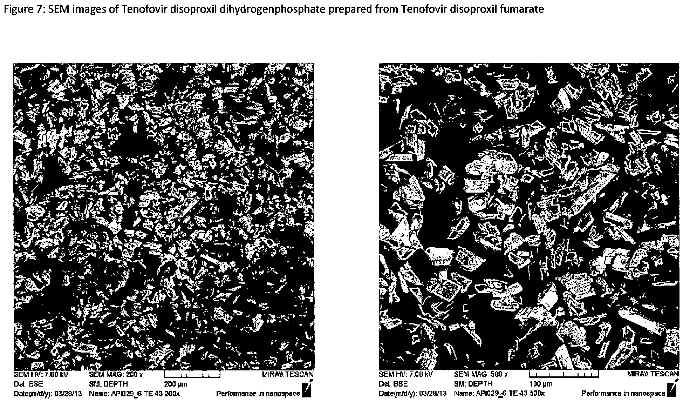

Tenofovir disoproxil fumarate (50 g) was dissolved in isopropanol (450 ml) in the 1 L vessel at 60 °C. The solution was seeded by the product (3 g). Phosphoric acid (6 ml of 85 % aq. phosphoric acid diluted by 50 ml isopropanol) was added dropwise during 30 min into the vessel. After that the suspension was cooled down to 20 °C by the cooling rate 0.3333 K/min. Tenofovir disoproxil phosphate was filtered, washed up two times by isopropanol and dried up (40 °C / 100 mbar / 16 hours). The isolated yield of crystallization was 90 % (HPLC: 99.76, Figure 6). The present of fumaric acid was <0.10 % (HPLC). Particle size distribution was obtained by scanning electron microscope. Most of the particles were smaller than 100 µm (Figure 7).

### Example 3:

### Preparation of drug product

Tenofovir disoproxil dihydrogenphosphate (300 g), lactose monohydrate (206 g), microcrystalline cellulose (Avicel pH 101; 50 g), croscarmellose sodium (30 g), polyvinylpyrolidone K-25 (20 g) and magnesium stearate (10 g) were sieved. The mixture was loaded to high shear granulator. Purified water (150 ml) was sprayed on the blend in short time intervals and the mixture was kneaded to granules. The wet granules were dried in an oven at 60°C. The dried granules were milled and sieved. The final mixture is compressed to tablets in the rotary tabletting machine.

### Description of drawings

Figure 1: DSC of Tenofovir disoproxil dihydrogenphosphate
Figure 2:1H NMR of of Tenofovir disoproxil dihydrogenphosphate
Figure 3: The apparent solubility and dissolution rate of Tenofovir disoproxil salts
Figure 4: Typical HPLC chromatogram of Tenofovir disoproxil dihydrogenphosphate prepared from Tenofovir disoproxil base
Figure 5: SEM images of Tenofovir disoproxil dihydrogenphosphate prepared from Tenofovir disoproxil
Figure 6: Typical HPLC chromatogram of Tenofovir disoproxil dihydrogenphosphate prepared from Tenofovir disoproxil fumarate
Figure 7: SEM images of Tenofovir disoproxil dihydrogenphosphate prepared from Tenofovir disoproxil fumarate

## Claims

1. A method of preparation of Tenofovir disoproxil dihydrogenphosphate of formula IV 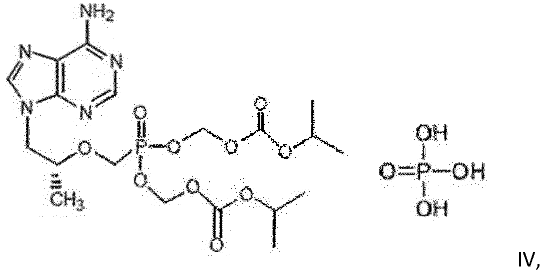 showing the following characteristic reflections in the XRPD measured by CuKα emission 5,6; 7,7; 12,4; 13,6; 16,4; 22,4 a 25,4 ± 0,2 ° 2θ, wherein Tenofovir disoproxil fumarate is dissolved in an organic solvent selected from C1-C4 alkylalcohols or toluene at temperature 55-60 °C, the solution is seeded by the product, phosphoric acid is added dropwise into the solution, the resulting reaction mixture is cooled down to 20-45 °C by the cooling ramp 0.3333 K/min and crystalline Tenofovir disoproxil dihydrogenphosphate is isolated.

2. A method of preparation according to claim 1, **characterized in that** Tenofovir disoproxil fumarate is dissolved in isopropanol at 60 °C, the solution is seeded by the product (5-10% of an amount of starting material), 1.1-5 equivalents of phosphoric acid (85 %) is added dropwise into the solution, the resulting reaction mixture is cooled down to 20 °C by the cooling ramp 0.3333 K/min and crystalline Tenofovir disoproxil dihydrogenphosphate is isolated.

## Patentansprüche

1. Verfahren zur Herstellung von Tenofovirdisoproxildihydrogenphosphat der Formel IV 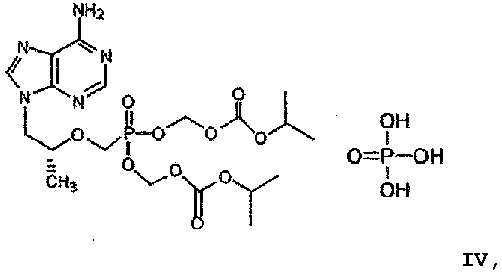 welches die folgenden charakteristischen Reflexe in der XRPD zeigt, gemessen durch CuKα-Strahlung 5,6; 7,7; 12,4; 13,6; 16,4; 22,4 und 25,4 ± 0,2 ° 2θ, wobei Tenofovirdisoproxilfumarat in einem organischen Lösungsmittel, ausgewählt aus C1-C4-Alkylalkoholen oder Toluol, bei einer Temperatur von 55 bis 60 °C gelöst wird, die Lösung mit dem Produkt geimpft wird, Phosphorsäure tropfenweise zu der Lösung gegeben wird, die resultierende Reaktionsmischung mit der Kühlrampe 0,3333 K/min auf 20-45 °C abgekühlt und kristallines Tenofovirdisoproxildihydrogenphosphat isoliert wird.

2. Verfahren zur Herstellung nach Anspruch 1, **dadurch gekennzeichnet, dass** Tenofovirdisoproxilfumarat bei 60 °C in Isopropanol gelöst wird, die Lösung mit dem Produkt (5-10 % einer Menge Ausgangsmaterial) geimpft wird, 1,1-5 Äquivalente Phosphorsäure (85 %) tropfenweise zu der Lösung gegeben wird, die resultierende Reaktionsmischung mit der Kühlrampe 0,3333 K/min auf 20 °C abgekühlt und kristallines Tenofovirdisoproxildihydrogenphosphat isoliert wird.

## Revendications

1. Un procédé de préparation du dihydrogénophosphate de ténofovir disoproxil de formule IV: 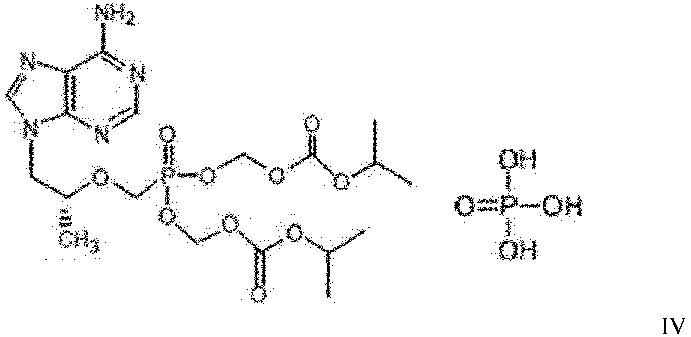 montrant dans la XRPD mesurées par l'émission de CuKα les réflexions caractéristiques suivantes 5,6; 7,7; 12,4; 13,6; 16,4; 22,4 a 25,4 ± 0,2 ° 2θ, dans lequel:
- le fumarate de ténofovir disoproxil est dissous dans un solvant organique choisi parmi les alcools alkyliques en C₁-C₄ ou le toluène, à une température de 55-60°C,
- la solution est ensemencée par le produit,
- l'acide phosphorique est ajouté goutte à goutte dans la solution,
- le mélange réactionnel résultant est refroidi à 20-45°C avec la rampe de refroidissement de 0,3333 K/min
- puis le dihydrogénophosphate de ténofovir disoproxil cristallin est isolé.

2. Un procédé de préparation selon la revendication 1, **caractérisé en ce que**:
- le fumarate de ténofovir disoproxil est dissous dans l'isopropanol à 60°C,
- la solution est ensemencée par le produit (5-10% d'une quantité de matière première),
- 1,1-5 équivalents d'acide phosphorique (85%) sont ajoutés goutte à goutte dans la solution,
- le mélange réactionnel résultant est refroidi à 20°C avec la rampe de refroidissement de 0,3333 K/min
- puis le dihydrogénophosphate de ténofovir disoproxil cristallin est isolé.
